(19) **Europäisches Patentamt European Patent Office Office européen des brevets**

(11) **EP 3 025 675 B1**

(12) **EUROPEAN PATENT SPECIFICATION**

(45) Date of publication and mention of the grant of the patent:
**03.10.2018 Bulletin 2018/40**

(51) Int Cl.:
*A61B 90/00* (2016.01)    *A61B 1/00* (2006.01)
*B25J 3/00* (2006.01)    *B25J 18/06* (2006.01)

(21) Application number: **14830359.7**

(86) International application number:
**PCT/JP2014/068754**

(22) Date of filing: **15.07.2014**

(87) International publication number:
**WO 2015/012150 (29.01.2015 Gazette 2015/04)**

(54) **MANIPULATOR SYSTEM**

MANIPULATORSYSTEM

SYSTÈME DE MANIPULATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2013   JP 2013155882**

(43) Date of publication of application:
**01.06.2016   Bulletin 2016/22**

(73) Proprietor: **Olympus Corporation Tokyo 192-8507 (JP)**

(72) Inventors:
• **HATAKEYAMA, Naoya Tokyo 192-8507 (JP)**
• **WAKAI, Hiroshi Tokyo 192-8507 (JP)**
• **IIDA, Masatoshi Tokyo 192-8507 (JP)**

(74) Representative: **Gunzelmann, Rainer Wuesthoff & Wuesthoff Patentanwälte PartG mbB Schweigerstraße 2 81541 München (DE)**

(56) References cited:
**WO-A1-2010/151438    JP-A- 2007 089 808
JP-A- 2008 245 840    JP-A- 2009 000 500
JP-A- 2010 214 128    US-A1- 2008 051 629**

**Description**

{Technical Field}

The present invention relates to a manipulator system.

{Background Art}

In general, an insertion portion of an endoscope has a treatment-tool channel formed therethrough in the longitudinal direction, allowing a treatment tool required for treatment for a patient to be guided to the inside of the body through the channel. Furthermore, the treatment tool in the channel can be moved forward/backward and rotated when an operator operates an operation instruction device (for example, see Patent Literature PTL 1 below).

A system, using an electric motor, for pushing/pulling the base end of the treatment tool in the longitudinal direction and for rotating the base end of the treatment tool in the circumferential direction is adopted as a mechanism for moving the treatment tool forward/backward and rotating the treatment tool. By rotating the motor by an amount proportional to an operation level input to the operation instruction device, the treatment tool can be moved forward/backward and rotated by the amount corresponding to the operation level.

In practice, however, the forward/backward movement and the rotary movement given to the base end of the treatment tool are attenuated while being transferred to the distal end of the treatment tool due to the friction produced between the treatment tool and an inner surface of the channel, the slack of the treatment tool in the channel, and the like. Specifically, the forward/backward amount and the rotation amount given to the base end of the treatment tool by the motor and a forward/backward amount and a rotation amount at the distal end of the treatment tool have a non-linear relationship. Furthermore, this non-linearity changes depending on the bending shape of the insertion portion. Therefore, if the rotation amount of the motor is made simply to be proportional to the operation level, it is impossible to obtain superior, uniform responsiveness of the treatment tool with respect to the operation by the operator. Thus, PTL 1 seeks to improve the responsiveness by controlling the motor so as to compensate for a reduction and a variation in the responsiveness of the bending section on the basis of the bending shape of the bending section, which is a factor that reduces the movement responsiveness of the treatment tool.

{Citation List}

{Patent Literature}

{PTL 1} Japanese Unexamined Patent Application, Publication No.2007-89808 Documents WO2010151438, US2008051629 and JP2008245840 also disclose multiple working channels in an endoscope, tools or instruments inserted therein and control for the instruments.

{Summary of Invention}

{Technical Problem}

However, there are factors that reduce the movement responsiveness of the treatment tool, other than the above-described bending shape of the bending section. Therefore, there is a problem in that the improvement of the movement responsiveness of the treatment tool is limited if only the bending shape of the bending section is taken into account, as in PTL 1.

The present invention has been made in view of the above-described circumstances, and an object thereof is to provide a manipulator system capable of always obtaining superior, uniform responsiveness by compensating for a reduction and a variation in the movement responsiveness of the treatment tool with a high degree of accuracy.

{Solution to Problem}

In order to achieve the above-described object, the present invention provides the following solutions.

The present invention provides a manipulator system including: a manipulator that includes an elongated insertion portion having a plurality of channels formed therethrough in the longitudinal direction, a treatment tool that is inserted into one of the channels of the insertion portion, and a treatment-tool driving part that allows the treatment tool to perform at least one of a forward/backward movement and a rotary movement in the channel; an operation input unit to which an operator inputs an operation instruction for the treatment tool; a control unit that generates a control signal for driving the treatment-tool driving part, according to the operation instruction input to the operation input unit; a channel-in-use detecting part that detects, among the plurality of channels, the channel into which the treatment tool has been inserted; and a compensation-value setting unit that sets a compensation value for the control signal on the basis of the channel detected by the channel-in-use detecting part, in which the control unit compensates the control signal by using the compensation value set by the compensation-value setting unit and sends the compensated control signal to the treatment-tool driving part.

According to the present invention, when an operator inputs an operation instruction to the operation input unit, the control unit sends a control signal generated from the operation instruction to the treatment-tool driving part, thereby allowing the treatment tool to perform at least one of a forward/backward movement and a rotation movement corresponding to the operation instruction. Accordingly, for example, the treatment tool disposed inside the body through a channel of the insertion portion can be remotely controlled by using the operation input unit, which is disposed outside the body.

In this case, the control unit sends the control signal to the treatment-tool driving part after compensating the control signal by using a compensation value set by the compensation-value setting unit. The movement responsiveness of the treatment tool to the operation signal depends on the properties of each channel, for example, an inner diameter and a friction coefficient of an inner surface. The compensation value is set on the basis of the channel into which the treatment tool has been inserted, which is detected by the channel-in-use detecting part. Accordingly, it is possible to always obtain superior, uniform responsiveness by compensating for a reduction and a variation in the movement responsiveness of the treatment tool with a high degree of accuracy.

In the above-described invention, the manipulator system may further include a treatment-tool identifying part that identifies the treatment tool that has been inserted into the channel, in which the compensation-value setting unit may set the compensation value additionally on the basis of a dynamic property of the treatment tool identified by the treatment-tool identifying part.

By doing so, the responsiveness of the treatment tool additionally depends on the dynamic properties of the treatment tool. Therefore, the dynamic properties of the treatment tool are also reflected in the compensation value, thereby making it possible to compensate for a reduction and a variation in the responsiveness of the treatment tool with a higher degree of accuracy.

In the above-described invention, the insertion portion may include an elongated flexible section that has flexibility and a bendable bending section that is provided at a distal end of the flexible section; the manipulator system may further include a bending-section-shape detecting part that detects a bending shape of the bending section; and the compensation-value setting unit may set the compensation value additionally on the basis of the bending shape of the bending section detected by the bending-section-shape detecting part.

By doing so, the responsiveness of the treatment tool additionally depends on the bending shape of the bending section. Therefore, the bending shape of the bending section is also reflected in the compensation value, thereby making it possible to compensate for a reduction and a variation in the responsiveness of the treatment tool with a higher degree of accuracy.

In the above-described invention, the insertion portion may include an elongated flexible section that has flexibility and a bendable bending section that is provided at a distal end of the flexible section; the manipulator system may further include a flexible-section-shape detecting part that detects a bending shape of the flexible section; and the compensation-value setting unit may set the compensation value additionally on the basis of the bending shape of the flexible section detected by the flexible-section-shape detecting part.

By doing so, the responsiveness of the treatment tool additionally depends on the bending shape of the flexible section. Therefore, the bending shape of the flexible section is also reflected in the compensation value, thereby making it possible to compensate for a reduction and a variation in the responsiveness of the treatment tool with a higher degree of accuracy.

In the above-described invention, the manipulator system may further include an external-section-shape detecting part that detects a bending shape of a base-end portion of the treatment tool, the base-end portion being pulled out from the insertion portion to the outside, in which the compensation-value setting unit may set the compensation value additionally on the basis of the bending shape of the base-end portion of the treatment tool detected by the external-section-shape detecting part.

By doing so, the responsiveness of the treatment tool additionally depends on the bending shape of the base-end portion, which is located outside the insertion portion. Therefore, the bending shape of the base-end portion of the treatment tool is also reflected in the compensation value, thereby making it possible to compensate for a reduction and a variation in the responsiveness of the treatment tool with a higher degree of accuracy.

In the above-described invention, the channels may be made of tubes that are disposed in the insertion portion along the longitudinal direction; and the tubes may be fixed in position, in radial position, in the insertion portion.

By doing so, variations in the friction, the slack produced in the treatment tool, and the like, which cause a variation in the responsiveness of the treatment tool, depend on a treatment-tool pathway that passes through the insertion portion. The treatment-tool pathway is defined by fixing in position, in the radial direction, the tube in the insertion portion, thereby making it possible to reduce the variations in the friction and in the slack, thus improving the accuracy in compensating for a reduction and a variation in the responsiveness of the treatment tool, performed by using the compensation value.

In the above-described invention, the control unit may perform feedforward control or feedback control for the treatment-tool driving part; and the compensation-value setting unit may set, as the compensation value, a gain that is used for the feedforward control or the feedback control by the control unit.

By doing so, a reduction and a variation in the responsiveness of the treatment tool can be compensated for by using a simple control method.

In the above-described invention, the control unit may send, as the control signal, a control signal on which an offset signal is superimposed, to the treatment-tool driving part; and the compensation-value setting unit may set, as the compensation value, the offset signal, whose sign is reversed when the direction of movement of the treatment tool is switched to the reverse direction.

By doing so, a backlash that occurs when the direction of movement of the treatment tool is reversed can be effectively resolved.

{Advantageous Effects of Invention}

According to the present invention, an advantageous effect is afforded in that it is possible to always obtain superior, uniform responsiveness by compensating for a reduction and a variation in the movement responsiveness of a treatment tool with a high degree of accuracy.

{Brief Description of Drawings}

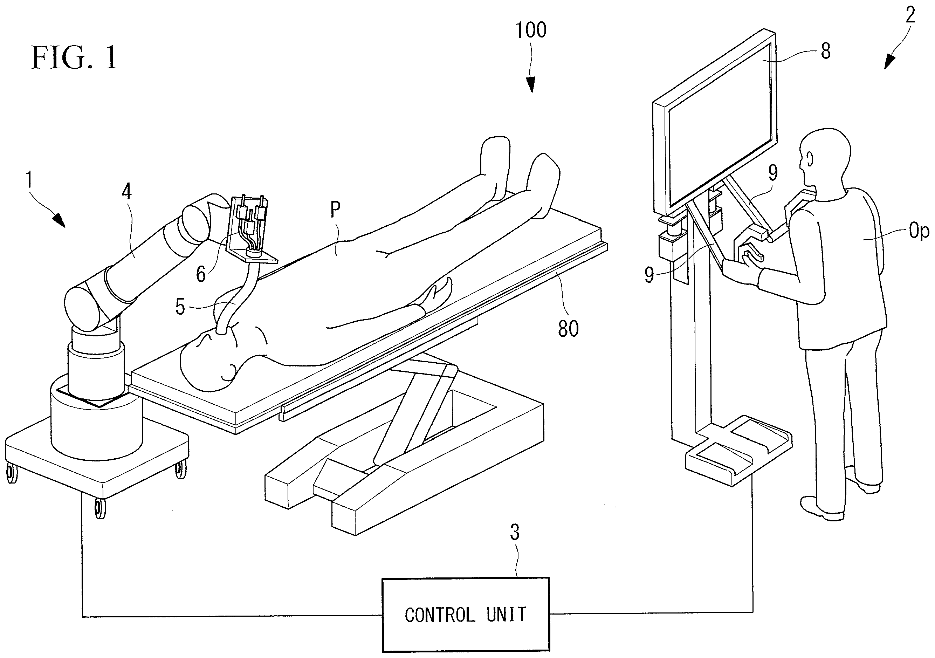

{Fig. 1} Fig. 1 is an appearance diagram showing the basic configuration of a manipulator system according to a first embodiment of the present invention.

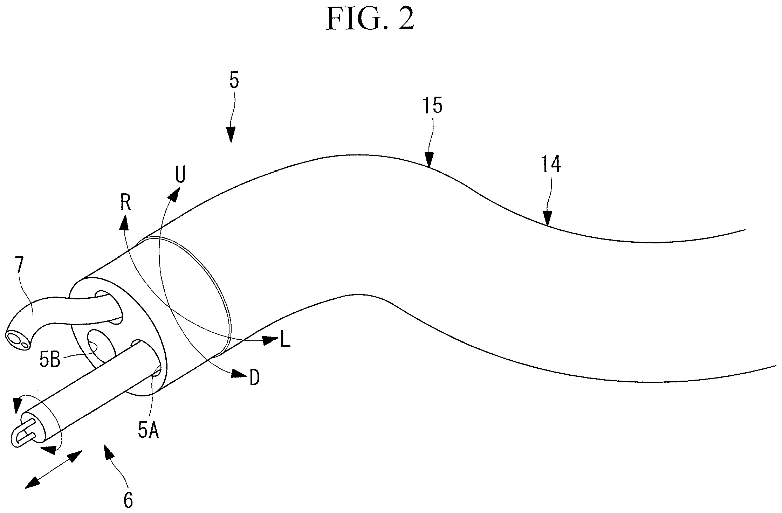

{Fig. 2} Fig. 2 is an appearance diagram showing the configuration of a distal end of an insertion portion provided in the manipulator system shown in Fig. 1.

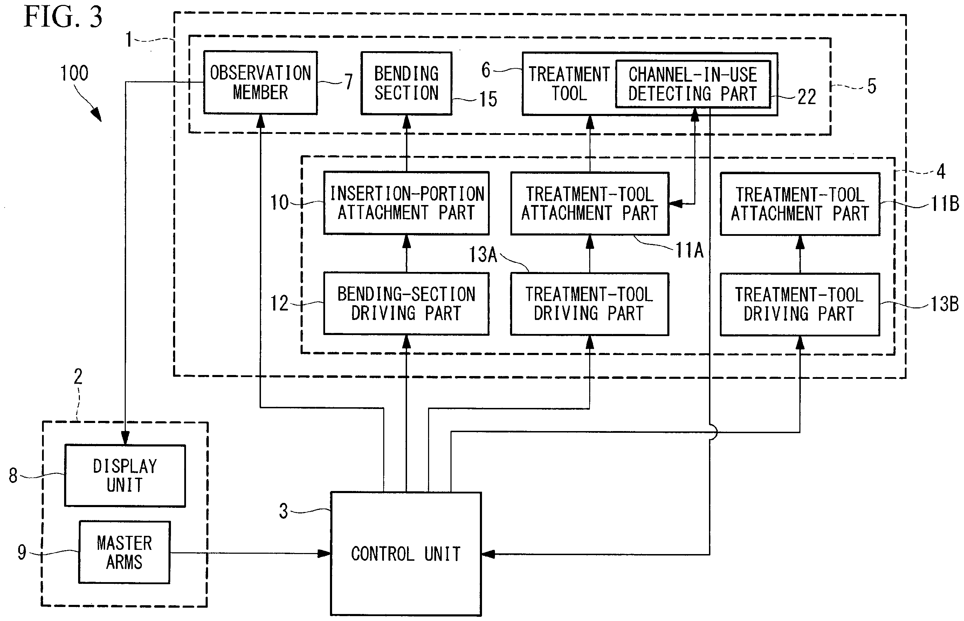

{Fig. 3} Fig. 3 is a block diagram showing the entire configuration of the manipulator system shown in Fig. 1.

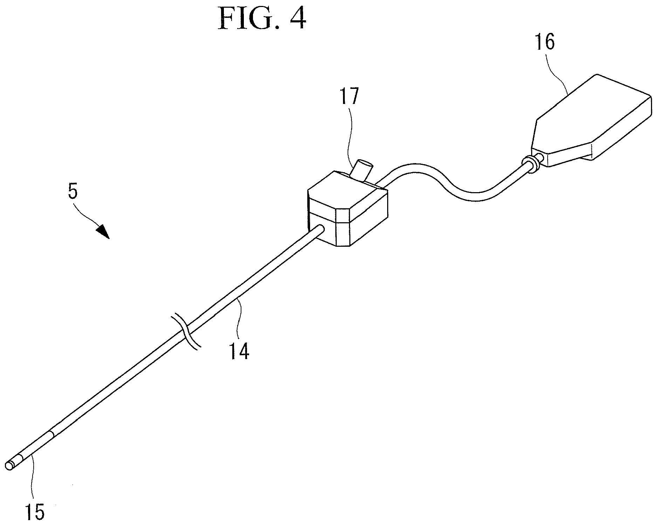

{Fig. 4} Fig. 4 is an appearance diagram showing the entire configuration of the insertion portion provided in the manipulator system shown in Fig. 1.

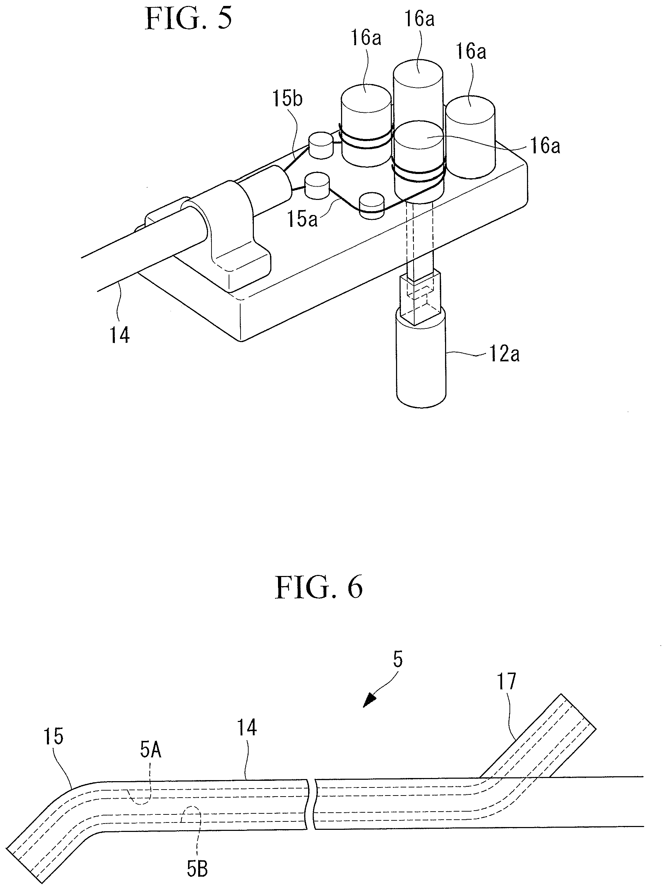

{Fig. 5} Fig. 5 is a structural diagram showing the mechanism for bending a bending section shown in Fig. 4.

{Fig. 6} Fig. 6 is a lateral view of the insertion portion, showing the structures of treatment-tool channels.

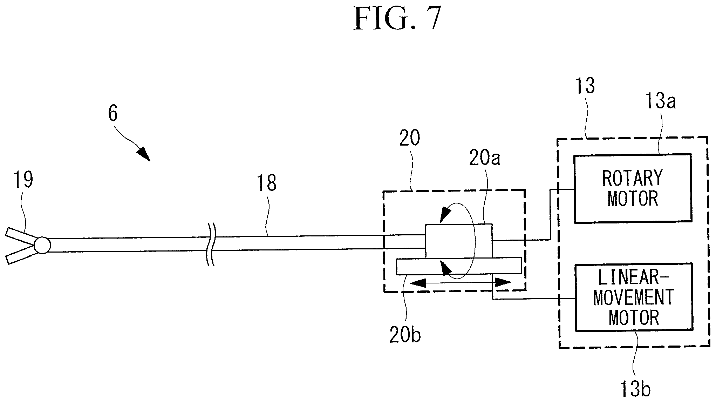

{Fig. 7} Fig. 7 is a view showing the entire configuration of a treatment tool provided in the manipulator system shown in Fig. 1.

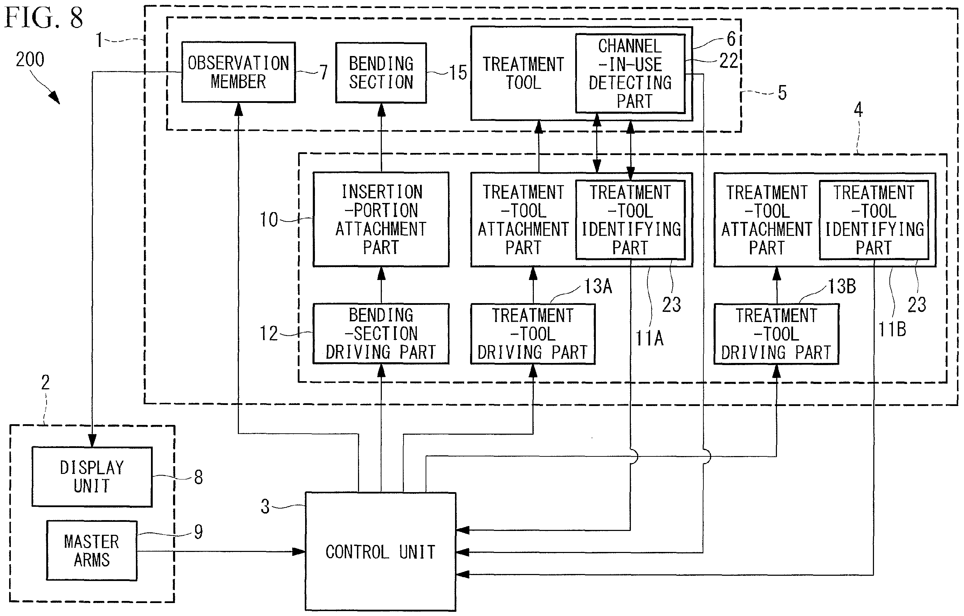

{Fig. 8} Fig. 8 is a block diagram showing the entire configuration of a manipulator system according to a second embodiment of the present invention.

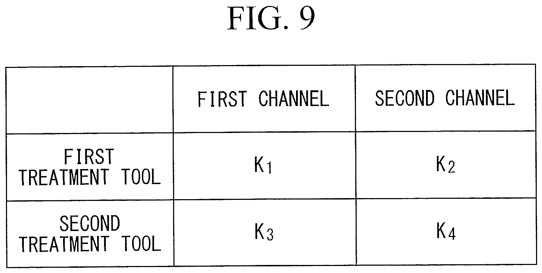

{Fig. 9} Fig. 9 is a table of FF gains held by a control unit shown in Fig. 8.

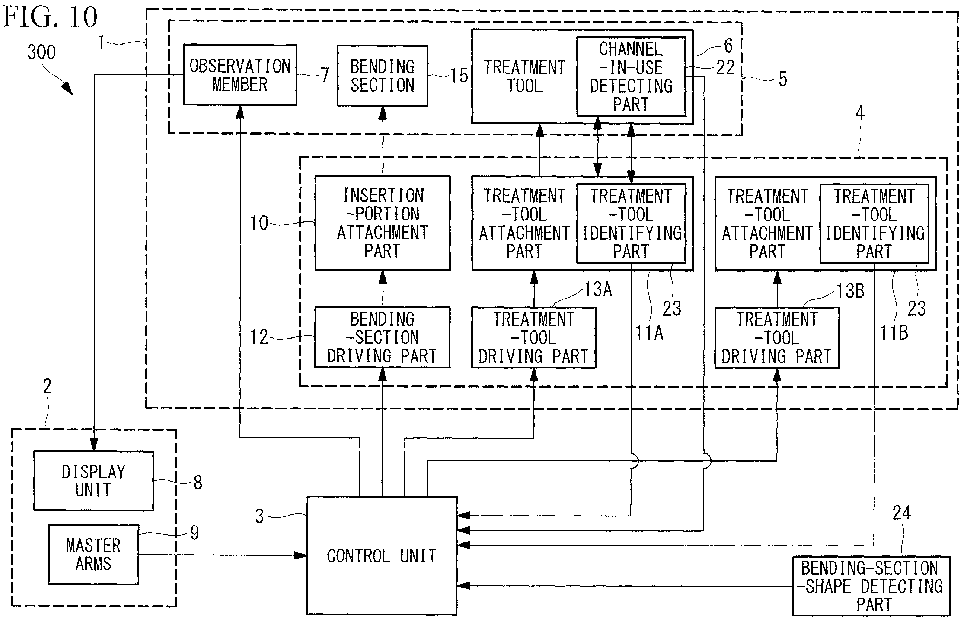

{Fig. 10} Fig. 10 is a block diagram showing the entire configuration of a manipulator system according to a third embodiment of the present invention.

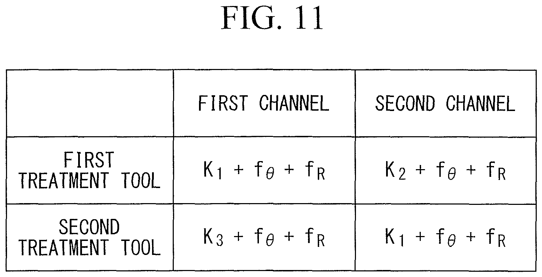

{Fig. 11} Fig. 11 is a table of FF gains held by a control unit shown in Fig. 10.

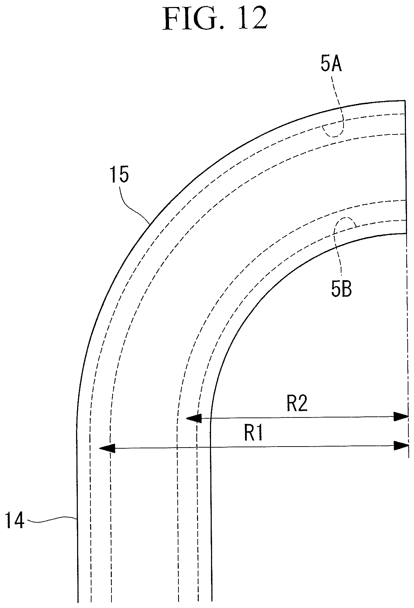

{Fig. 12} Fig. 12 is a lateral view of the bending section, for explaining the relationship between a bending angle of the bending section and bending radii of the channels.

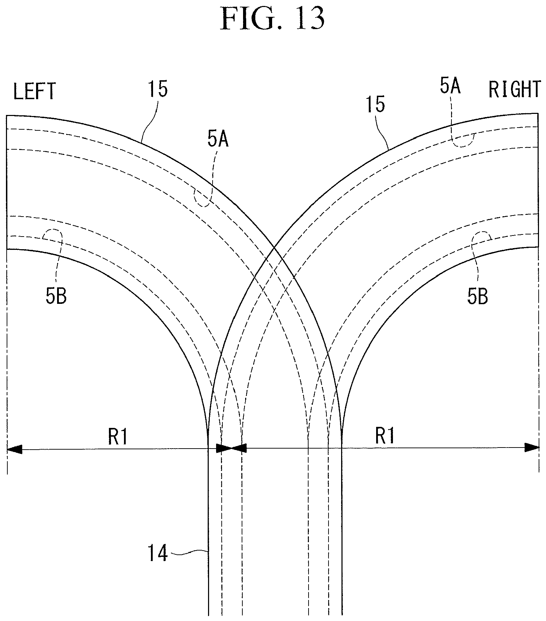

{Fig. 13} Fig. 13 is a lateral view of the bending section, for explaining the relationship between bending directions of the bending section and bending radii of each of the channels.

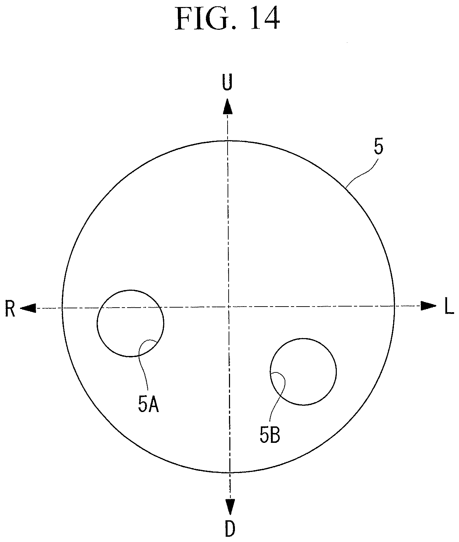

{Fig. 14} Fig. 14 is a front view of the insertion portion, for explaining the relationship between bending directions of the bending section and the positions of the two channels.

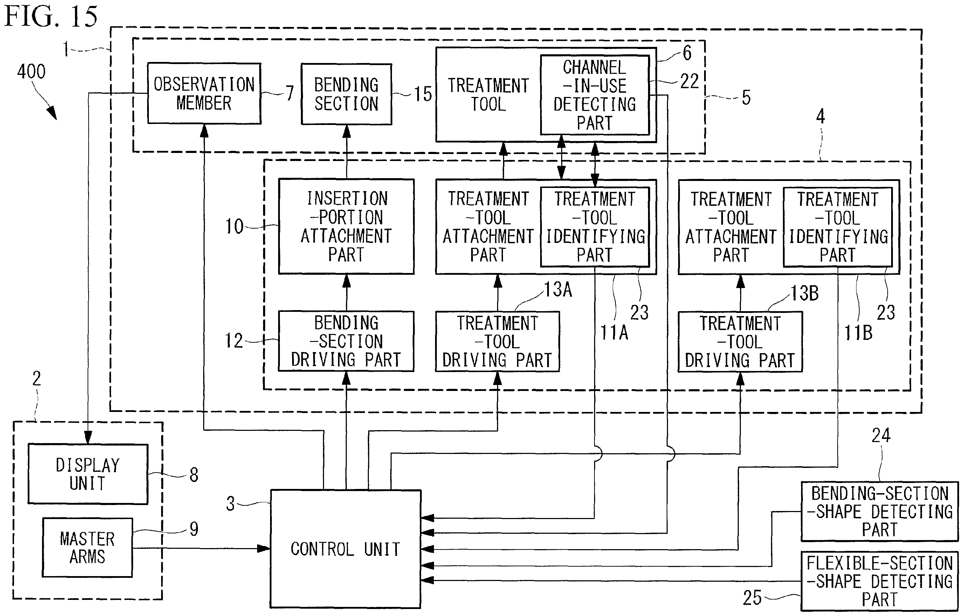

{Fig. 15} Fig. 15 is a block diagram showing the entire configuration of a manipulator system according to a fourth embodiment of the present invention.

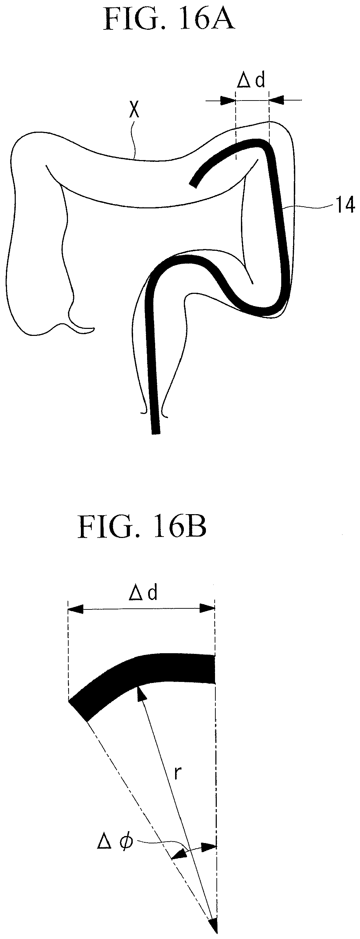

{Fig. 16A} Fig. 16A is a view for explaining a method of calculating a feature quantity k by using a flexible-section-shape detecting part shown in Fig. 15 and for showing a flexible-section bending shape detected by the flexible-section-shape detecting part.

{Fig. 16B} Fig. 16B is a view for explaining the method of calculating the feature quantity k by using the flexible-section-shape detecting part shown in Fig. 15 and for showing variables in a small segment.

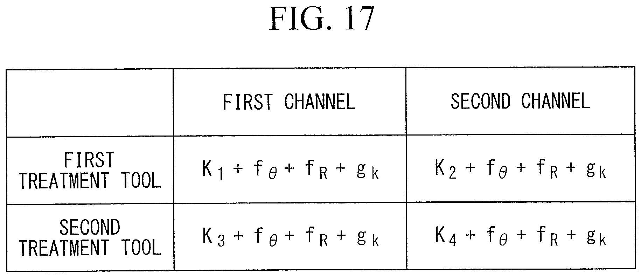

{Fig. 17} Fig. 17 is a table of FF gains held by a control unit shown in Fig. 15.

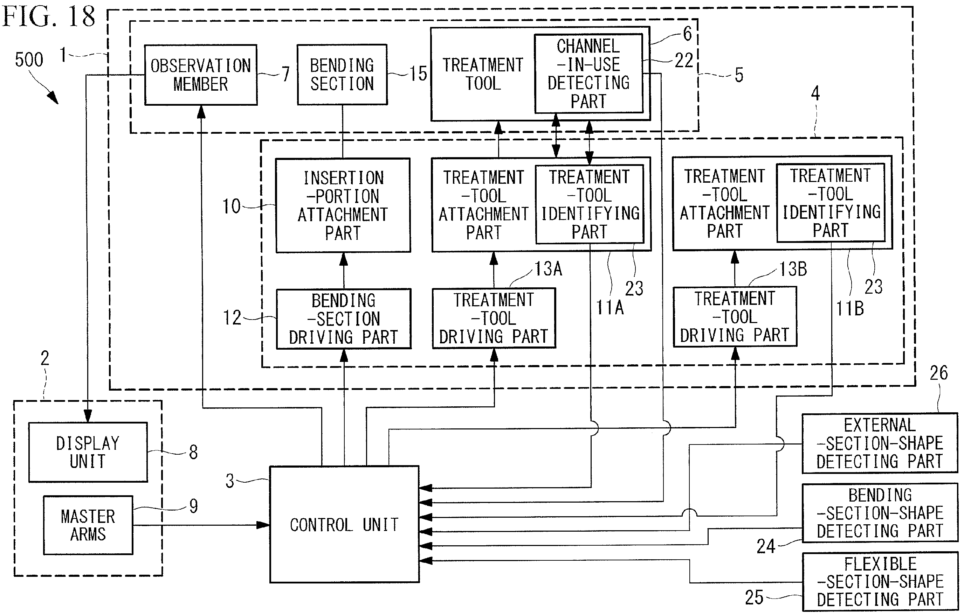

{Fig. 18} Fig. 18 is a block diagram showing the entire configuration of a manipulator system according to a fifth embodiment of the present invention.

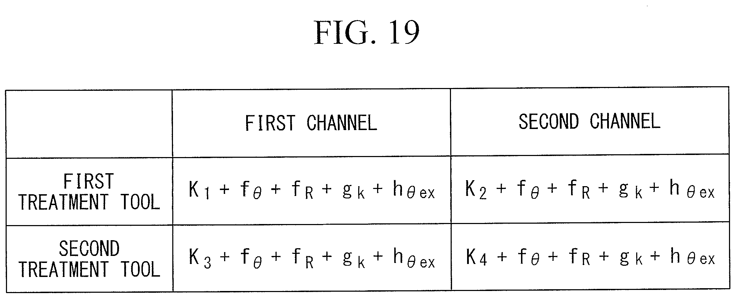

{Fig. 19} Fig. 19 is a table of FF gains held by a control unit shown in Fig. 18.

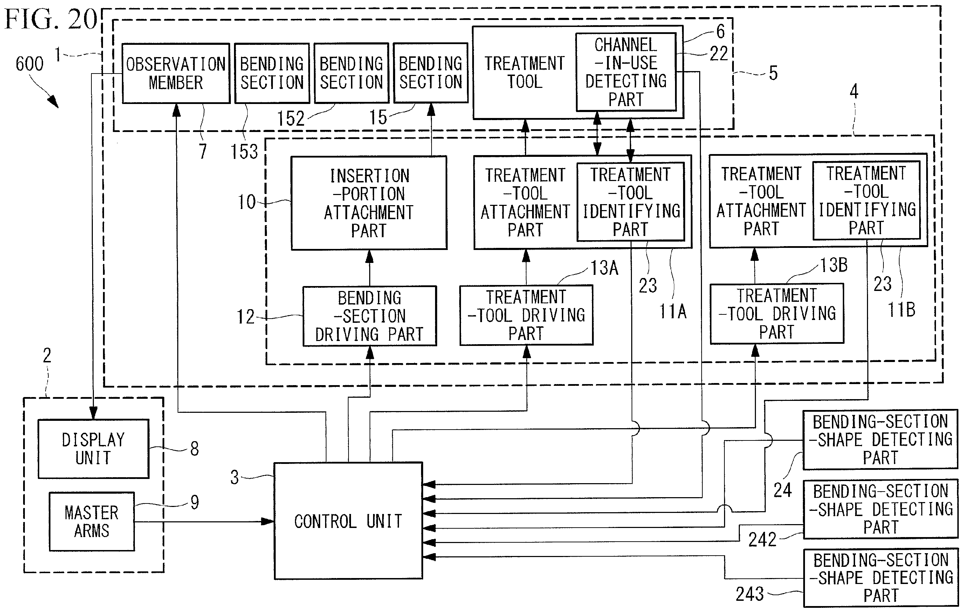

{Fig. 20} Fig. 20 is a block diagram showing the entire configuration of a manipulator system according to a sixth embodiment of the present invention.

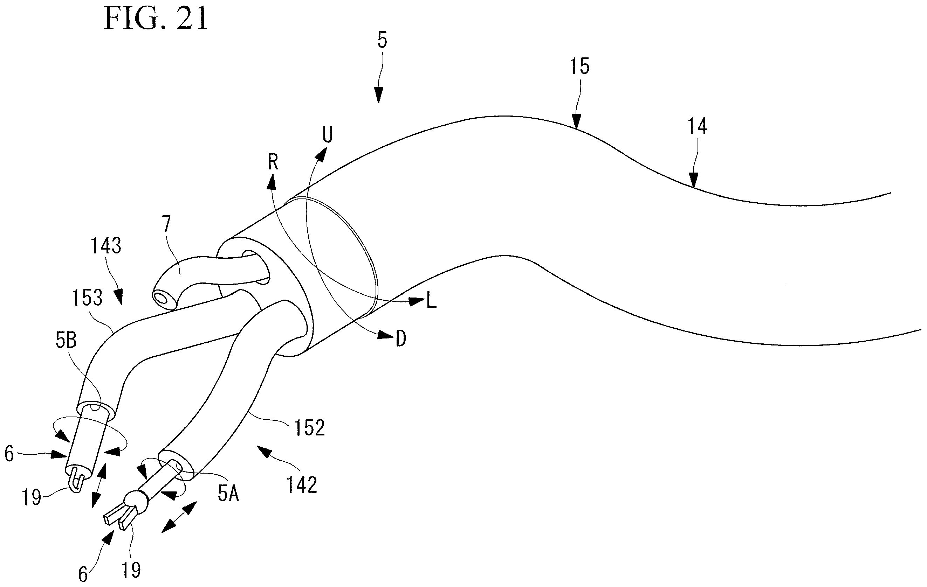

{Fig. 21} Fig. 21 is an appearance diagram showing the configuration of a distal end of an insertion portion provided in the manipulator system shown in Fig. 20.

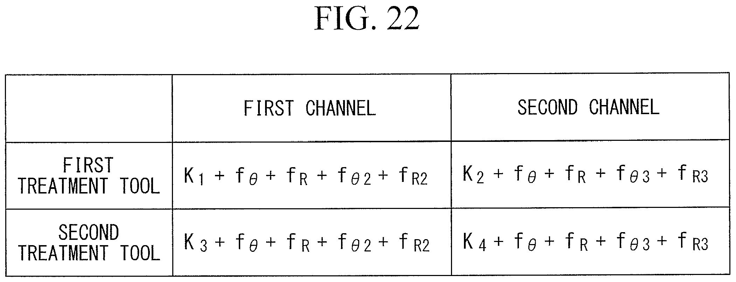

{Fig. 22} Fig. 22 is a table of FF gains held by a control unit shown in Fig. 20.

{Description of Embodiments}

First Embodiment

A manipulator system 100 according to a first embodiment of the present invention will be described with reference to Figs. 1 to 7.

First, the outline of the manipulator system 100 of this embodiment will be described. As shown in Fig. 1, the manipulator system 100 of this embodiment is provided with, as the basic configuration, a slave manipulator (manipulator) 1, a master input unit (operation input unit) 2 that is operated by an operator Op, and a control unit 3 that controls the slave manipulator 1 on the basis of an operation input to the master input unit 2.

The slave manipulator 1 is provided with a slave arm 4 that is disposed in the vicinity of an operating table 80 on which a patient P lies, an insertion portion 5 that is held at a distal end of the slave arm 4, and a treatment tool 6 that is inserted into the insertion portion 5. As shown in Fig. 2, an observation member 7 is provided at a distal end of the insertion portion 5 so as to image a visual field in front of the distal end of the insertion portion 5 and the treatment tool 6 protruding from the distal end of the insertion portion 5. Video acquired by the observation member 7 is displayed on a display unit 8 that is provided in the master input unit 2. The visual field of the observation member 7 can be moved by changing the bending angle of a bending section 15 that is provided at a distal end portion of the insertion portion 5 in an up/down direction (UD direction) or a right/left direction (LR direction), which is perpendicular to the longitudinal direction of the insertion portion 5.

The operator Op operates master arms 9 that are provided in the master input unit 2 while observing a video of the inside of the body and the treatment tool 6 displayed on the display unit 8, thereby making it possible to remotely operate the insertion portion 5, inserted into the body of the patient P, and the treatment tool 6, introduced to the inside of the body through the insertion portion 5.

Next, components of the manipulator system 100 will be described in detail.

As shown in Fig. 3, the slave arm 4 is provided with an insertion-portion attachment part 10 to which the insertion portion 5 is attached, a plurality of treatment-tool attachment parts 11A and 11B to either of which the treatment tool 6 is attached, a bending-section driving part 12 that drives the bending section 15 of the insertion portion 5 attached to the insertion-portion attachment part 10, and a plurality of treatment-tool driving parts 13A and 13B that each drive the treatment tool 6 attached to the corresponding treatment-tool attachment part 11A or 11B. In this embodiment, as will be described later, on the assumption that the insertion portion 5 is provided with two channels 5A and 5B into either of which the treatment tool 6 is inserted, the two treatment-tool attachment parts 11A and 11B and the two treatment-tool driving parts 13A and 13B are provided for the channels 5A and 5B, respectively. Hereinafter, the two treatment-tool attachment parts 11A and 11B are also simply referred to as a treatment-tool attachment part 11. Furthermore, the two treatment-tool driving parts 13A and 13B are also simply referred to as a treatment-tool driving part 13.

Fig. 4 shows the appearance of the insertion portion 5. As shown in Fig. 4, the insertion portion 5 has an elongated flexible section 14 having flexibility and the bendable bending section 15, which is provided at a distal end of the flexible section 14. An attachment unit 16 to be attached to the insertion-portion attachment part 10 of the slave arm 4 is connected to a base end of the flexible section 14. The bending section 15 has a known structure in which a plurality of nodal rings or curved comas are connected. The bending section 15 is configured so as to be bent in the UD direction and in the LR direction by pushing/pulling, in the attachment unit 16, base ends of a UD-bending wire 15a and an LR-bending wire 15b that are connected to the nodal ring or the like that is located closest to the distal end.

Specifically, as shown in Fig. 5, the base ends of the wires 15a and 15b are pulled out from the base end of the flexible section 14 and are wound up by pulleys 16a provided in the attachment unit 16. The attachment unit 16 is configured such that, when attached to the insertion-portion attachment part 10, the pulleys 16a are coaxially connected to motors 12a that are included in the bending-section driving part 12. When the motors 12a are rotated in response to bending control signals sent from the control unit 3, the pulleys 16a are rotated clockwise or counterclockwise, thereby pushing or pulling the wires 15a and 15b and changing the bending angle of the bending section 15. Note that, in order to simplify the figure, Fig. 5 shows only one motor 12a and also only the two wires 15a and 15b wound up by two pulleys 16 that are located closer to the flexible section 14.

As shown in Fig. 6, the insertion portion 5 has a plurality of (in this example, two) channels 5A and 5B that are formed therethrough from the distal end of the insertion portion 5 along the longitudinal direction. The channels 5A and 5B are made of flexible tubes and extend from the distal end of the insertion portion 5 to a treatment-tool port 17 that is provided close to the base end of the insertion portion 5. The treatment tool 6 is inserted into each channel 5A or 5B from the treatment-tool port 17.

The treatment tool 6 is a high-frequency knife, a snare loop, or a pair of grasper forceps and, as shown in Fig. 7, is provided with an elongated main body 18 that has flexibility and a treatment part 19 that is provided at a distal end of the main body 18. An attachment unit 20 that is to be attached to the treatment-tool attachment part 11 of the slave arm 4 is connected to a base end of the main body 18. The base end of the main body 18 is pushed/pulled in the attachment unit 20 in the longitudinal direction or is rotated therein in the circumferential direction, thereby moving forward/backward or rotating the entire treatment tool 6 in the channel 5A or 5B.

Specifically, a pulley 20a that is coaxially connected to the base end of the main body 18 is provided in the attachment unit 20. The pulley 20a is fixed on a stage 20b that can be linearly moved in the longitudinal direction of the main body 18. Meanwhile, the treatment-tool driving part 13 is provided with a rotary motor 13a that rotates the pulley 20a and a linear-movement motor 13b that linearly moves the stage 20b. The attachment unit 20 is configured such that, when attached to the treatment-tool attachment part 11, the pulley 20a and the rotary motor 13a are coupled, and the stage 20b and the linear-movement motor 13b are coupled. Accordingly, the rotary motor 13a rotates the pulley 20a in response to a rotary control signal (control signal) sent from the control unit 3, thereby rotating the treatment tool 6. Furthermore, the linear-movement motor 13b linearly moves the stage 20b in response to a forward/backward control signal (control signal) sent from the control unit 3, thereby moving the treatment tool 6 forward/backward.

As described above, the master input unit 2 is provided with the display unit 8, which displays a video acquired by the observation member 7, and the plurality of master arms 9, which are operated by the operator Op. The operator Op can input, to the master arms 9, operation instructions at least for the bending section 15 and the treatment tool 6. The master input unit 2 generates operation signals based on the operation instructions input to the master arms 9 by the operator Op and sends the generated operation signals to the control unit 3.

When receiving an operation signal for the bending section 15 from the master input unit 2, the control unit 3 generates, from the operation signal, a bending control signal for driving the bending-section driving part 12 and sends the bending control signal to the bending-section driving part 12. Furthermore, when receiving an operation signal for the treatment tool 6 from the master input unit 2, the control unit 3 generates, from the operation signal, a forward/backward control signal and a rotary control signal for driving the treatment-tool driving part 13 and sends the forward/backward control signal and the rotary control signal to the treatment-tool driving part 13. The motors 12a, 13a, and 13b of the driving parts 12 and 13 have encoders (not shown) attached thereto for detecting rotation amounts thereof. By receiving the rotation amounts of the motors 12a, 13a, and 13b from the encoders, the control unit 3 recognizes a bending amount of the bending section 15 and a forward/backward amount or a rotation amount of the treatment tool 6 and performs feedback (FB) control for the motors 12a, 13a, and 13b of the driving parts 12 and 13 on the basis of the recognized amounts.

Furthermore, the manipulator system 100 of this embodiment is provided with a channel-in-use detecting part 22 that detects the channel 5A or 5B into which the treatment tool 6 is inserted.

The treatment-tool attachment parts 11A and 11B are provided with recording media, such as barcodes or IC tags, that have recorded identification information for identifying the corresponding channels 5A and 5B. The channel-in-use detecting part 22 is provided in the attachment unit 20, reads identification information recorded in the recording medium of the treatment-tool attachment part 11A or 11B, when the attachment unit 20 is attached to the treatment-tool attachment part 11A or 11B, and sends the read identification information to the control unit 3 via the slave arm 4.

Note that the channel-in-use detecting part 22 may be configured so as to identify the channels 5A and 5B electrically or magnetically. For example, the treatment-tool attachment parts 11 may be provided with magnets or resistances having properties that are different for the corresponding channels 5A and 5B, and the channel-in-use detecting part 22 may be configured to detect the properties of the magnets or resistances. Alternatively, the channel-in-use detecting part 22 may be composed of an input means, such as a keyboard, a touch panel, or buttons.

Here, when receiving an operation signal for the treatment tool 6 from the master input unit 2, the control unit (compensation-value setting unit) 3 sets, before sending a forward/backward control signal and a rotary control signal to the treatment-tool driving part 13, a compensation value for the forward/backward control signal and the rotary control signal.

Specifically, the control unit 3 stores a compensation value for the forward/backward control signal and the rotary control signal in association with each of the channels 5A and 5B. The compensation value is a feedforward (FF) gain $K_1$ or $K_2$ by which the forward/backward control signal and the rotary control signal are multiplied. The compensation value is, for example, a value experimentally determined by measuring, when the channel 5A or 5B is used, the relationship between the forward/backward control signal and the rotary control signal input to the corresponding treatment-tool driving part 13A or 13B and an actual forward/backward distance and an actual rotation angle of the distal end of the treatment tool 6.

The control unit 3 recognizes the channel 5A or 5B into which the treatment tool 6 has been inserted on the basis of the identification information of the channel 5A or 5B received from the channel-in-use detecting part 22 and selects the FF gain $K_1$ or $K_2$ corresponding to the recognized channel 5A or 5B. Next, the control unit 3 multiplies the forward/backward control signal and the rotary control signal, which are generated from the operation signal, by the FF gain $K_1$ or $K_2$, thereby amplifying the forward/backward control signal and the rotary control signal, and sends the amplified forward/backward control signal and rotary control signal to the treatment-tool driving part 13A or 13B corresponding to the recognized channel 5A or 5B, thereby performing feedforward control for the treatment-tool driving part 13A or 13B.

Next, the operation of the thus-configured manipulator system 100 will be described.

In order to treat the inside of the body by using the manipulator system 100 of this embodiment, as shown in Fig. 1, the operator Op first inserts the insertion portion 5 into the body of the patient P from a natural orifice (in the example shown in the figure, the mouth). The operator Op moves the distal end of the insertion portion 5 up to an area to be treated, while observing, on the display unit 8, a video acquired by the observation member 7.

Next, the operator Op inserts the treatment tool 6 into the channel 5A or 5B selected depending on the treatment and attaches the attachment unit 20 to the treatment-tool attachment part 11A or 11B corresponding to the selected channel 5A or 5B. In this example, it is assumed that the treatment tool 6 is inserted into the first channel 5A, and the attachment unit 20 is attached to the first treatment-tool attachment part 11A. Identification information in the recording medium provided on the first treatment-tool attachment part 11A is read by the channel-in-use detecting part 22 and is sent to the control unit 3, thereby allowing the control unit 3 to recognize that the treatment tool 6 has been inserted into the first channel 5A. Then, the control unit 3 selects the FF gain $K_1$ corresponding to the first channel 5A.

Next, the operator Op makes the treatment tool 6 that has been inserted into the channel 5A protrude from an opening at the distal end of the insertion portion 5. Then, while observing the video displayed on the display unit 8, the operator Op changes the bending angle of the bending section 15 and the protrusion amount and the direction of rotation of the treatment tool 6, thereby adjusting the positional relationship between the treatment part 19 and the area to be treated in the body and treating the area by using the treatment part 19.

At this time, when the operator Op inputs, to the master arms 9, an operation for moving the treatment tool 6 forward or backward, an operation signal based on the operation is sent from the master arms 9 to the control unit 3. From the received operation signal, the control unit 3 generates a forward/backward control signal for moving the treatment tool 6 forward/backward and a rotary control signal for rotating the treatment tool 6. Next, the control unit 3 sends, to the treatment-tool driving part 13A, the forward/backward control signal and the rotary control signal that have been amplified by the FF gain $K_1$, thereby performing FF control for the treatment-tool driving part 13A.

In order to use the treatment tool 6 in the second channel 5B, the operator Op pulls the treatment tool 6 out from the first channel 5A and inserts the treatment tool 6 into the second channel 5B. At this time, the operator Op relocates and attaches the attachment unit 20 to the second treatment-tool attachment part 11B corresponding to the second channel 5B. Identification information in the recording medium provided on the second treatment-tool attachment part 11B is read by the channel-in-use detecting part 22 and is sent to the control unit 3, thereby allowing the control unit 3 to recognize that the treatment tool 6 has been moved from the first channel 5A to the second channel 5B. Thus, the control unit 3 changes from the FF gain $K_1$ to the FF gain $k_2$ corresponding to the second channel 5B and changes the destination of the forward/backward control signal and the rotary control signal to the treatment-tool driving part 13B.

Here, a description will be given of the movement response characteristics of the treatment tool 6 with respect to an operation input to the master arms 9 by the operator Op.

The linear movement and the rotary movement given to the base end of the main body 18 by the motors 13a and 13b are attenuated while being transferred to the distal end of the main body 18, due to factors such as the friction produced between the treatment tool 6 and an inner surface of the channel 5A or 5B and the slack of the main body 18 in the channel 5A or 5B. Accordingly, the actual forward/backward amount and rotation amount of the treatment part 19 are reduced with respect to the outputs of the motors 13a and 13b. The above-described friction and slack vary depending on the inner diameter of the channel 5A or 5B and the material of the inner surface thereof. As a result, even when the same treatment tool 6 is used, the movement responsiveness of the treatment tool 6 varies depending on whether the treatment tool 6 is used in the channel 5A or 5B.

According to this embodiment, the FF gain $K_1$ or $K_2$ for amplifying the forward/backward control signal and the rotary control signal is set according to the channel 5A or 5B, thereby compensating for a variation in the movement responsiveness of the treatment tool 6, which depends on the difference in properties between the channels 5A and 5B. Accordingly, regardless of whether the treatment tool 6 is used in either the channel 5A or 5B, it is possible to always obtain superior, uniform responsiveness of the forward/backward movement and the rotary movement of the treatment tool 6.

Note that, in this embodiment, although the compensation value is a feedforward gain, instead of this, another compensation value may be used.

For example, the compensation value may be a feedback gain that the control unit 3 uses for feedback control for the treatment-tool driving part 13.

As described above, the control unit 3 performs feedback control for the motors 12a, 13a, and 13b on the basis of the outputs from the motors 12a, 13a, and 13b, detected by the encoders. Thus, the control unit 3 may set a feedback gain for each of the channels 5A and 5B. By doing so, the variation in the responsiveness of the treatment tool 6, which is caused by the difference between the channels 5A and 5B, can be compensated for with a high degree of accuracy.

Alternatively, the compensation value may be a friction compensation coefficient (offset signal) to be added to the forward/backward control signal and the rotary control signal by the control unit 3 in order to offset the control signals. The friction compensation coefficient is set such that the sign is reversed at a turn-back point where the direction of the movement of the treatment tool 6 is switched to the reverse direction. Accordingly, it is possible to reduce a backlash that occurs particularly when the direction of movement is switched to the reverse direction (for example, when the left rotation is switched to the right rotation).

Second Embodiment

Next, a manipulator system 200 according to a second embodiment of the present invention will be described with reference to Figs. 8 and 9.

As shown in Fig. 8, the manipulator system 200 of this embodiment differs from that of the first embodiment in that the manipulator 1 is provided with two treatment tools 6 and also treatment-tool identifying parts 23 that identify the treatment tools 6 inserted into the channels 5A and 5B. Therefore, in this embodiment, the treatment-tool identifying parts 23 will be mainly described, identical reference signs will be assigned to the same configurations as those in the first embodiment, and a description thereof will be omitted.

The two treatment tools 6 differ in type, for example, and also differ in the stiffness of the main body 18 and in the friction coefficient of the surface. Note that the manipulator 1 may be provided with three or more treatment tools 6. Note that Fig. 8 shows only a first treatment tool 6 inserted into the channel 5A and does not show a second treatment tool.

In this embodiment, the attachment unit 20 is provided with a recording medium, such as a barcode or an IC tag. This recording medium has recorded identification information for identifying the treatment tool 6 to which the attachment unit 20 has been connected. The treatment-tool identifying parts 23 are provided in the treatment-tool attachment parts 11. When the attachment unit 20 is attached to each of the treatment-tool attachment parts 11, the corresponding treatment-tool identifying part 23 reads the identification information of the treatment tool 6 from the recording medium provided on the attachment unit 20 and sends the read identification information of the treatment tool 6 to the control unit 3.

The control unit 3 recognizes that either of the treatment tools 6 has been inserted into the channel 5A or 5B on the basis of the identification information of the treatment tool 6 received from the treatment-tool identifying part 23. The control unit 3 sets an FF gain according to the identification information of the channel 5A or 5B, received from the channel-in-use detecting part 22, and the identification information of the treatment tool 6, received from the treatment-tool identifying part 23. Specifically, as shown in Fig. 9, the control unit 3 has a table in which the channels 5A and 5B and the treatment tools 6 are associated with FF gains $K_1$, $K_2$, $K_3$, and $K_4$. The control unit 3 selects, from among the four FF gains $K_1$, $K_2$, $K_3$, and $K_4$, the FF gain that is associated with the combination of the channel 5A or 5B and the treatment tool 6, which are identified by the identification information.

Next, the operation of the thus-configured manipulator system 200 will be described.

The basic procedure is the same as that in the first embodiment. The manipulator system 200 of this embodiment differs from that of the first embodiment in the FF-gain setting method.

The operator Op inserts the treatment tool 6 that is selected for the treatment into the channel 5A or 5B and attaches the attachment unit 20 to the treatment-tool attachment part 11A or 11B corresponding to the channel 5A or 5B into which the treatment tool 6 has been inserted. For example, it is assumed that the first treatment tool 6 is inserted into the first channel 5A. The identification information of the channel 5A, which is provided on the first treatment-tool attachment part 11A, is read by the channel-in-use detecting part 22 and is sent to the control unit 3. At the same time, the identification information of the treatment tool 6, which is provided thereon, is read by the treatment-tool identifying part 23 and is sent to the control unit 3. Accordingly, the control unit 3 recognizes that the first treatment tool 6 has been inserted into the first channel 5A. The control unit 3 selects the FF gain $K_1$, which is set for the combination of the first channel 5A and the first treatment tool 6. After that, when an operation for the treatment tool 6 is input to the master arms 9, the control unit 3 multiplies the forward/backward control signal and the rotary control signal by the FF gain $K_1$ and sends the amplified control signals to the treatment-tool driving part 13A.

When the operator Op changes the treatment tool 6 or the channel 5A or 5B, the control unit 3 newly receives identification information from the channel-in-use detecting part 22 and the treatment-tool identifying part 23 and switches from the FF gain $K_1$ to the FF gain $K_2$, $K_3$, or $K_4$ on the basis of the received identification information.

Here, the above-described movement responsiveness of each treatment tool 6 varies depending, in addition, on the combination of the channel 5A or 5B and the treatment tool 6. Specifically, the friction produced between the treatment tool 6 and the inner surface of the channel 5A or 5B varies depending on the combination of the inner diameter and the material of the inner surface of the channel 5A or 5B and the outer shape and the material of the treatment tool 6. Furthermore, the responsiveness varies for each treatment tool 6 depending on the dynamic properties, such as the stiffness, of the main body 18 of the treatment tool 6. As a result, even if the same channel 5A or 5B is used, when the treatment tool 6 to be inserted into that channel 5A or 5B is different, the movement responsiveness varies for each treatment tool 6.

According to this embodiment, the FF gain $K_1$, $K_2$, $K_3$, or $K_4$ is set depending, in addition, on the combination of the channel 5A or 5B and the treatment tool 6, thereby further compensating for a variation in the movement responsiveness of the treatment tool 6, which varies depending on the combination of the channel 5A or 5B and the treatment tool 6. Accordingly, regardless of whether any treatment tool 6 is used in the channel 5A or 5B, it is possible to always obtain superior, uniform responsiveness of the forward/backward movement and the rotary movement of the treatment tool 6.

Third Embodiment

Next, a manipulator system 300 according to a third embodiment of the present invention will be described with reference to Figs. 10 to 14.

As shown in Fig. 10, the manipulator system 300 of this embodiment is obtained by further providing, in the manipulator system 200 of the second embodiment, a bending-section-shape detecting part 24 that detects a bending angle θ of the bending section 15. Therefore, in this embodiment, the bending-section-shape detecting part 24 will be mainly described, identical reference signs will be assigned to the same configurations as those in the first and second embodiments, and a description thereof will be omitted.

The bending-section-shape detecting part 24 receives the rotation angles of the rotary motors 12a from the encoders provided in the rotary motors 12a of the bending-section driving part 12 and converts the received rotation angles into the bending angle θ of the bending section 15, thus obtaining the bending angle θ.

Note that the bending-section-shape detecting part 24 may detect the bending angle of the bending section 15 by using another means, instead of the above-described outputs of the encoders. For example, the bending-section-shape detecting part 24 may detect the bending angle θ of the bending section 15 on the basis of a bending control signal sent from the control unit 3 to the bending-section driving part 12. Alternatively, the bending-section-shape detecting part 24 may detect an actual bending angle of the bending section 15 by using bending sensors that are provided on the bending section 15 at a plurality of positions in the longitudinal direction. As the bending sensors, for example, distortion sensors, optical fiber sensors, etc., are used. Furthermore, it is also possible to provide linear members that are fixed at the distal ends of the wires 15a and 15b, that extend up to the base end of the flexible section 14 parallel to the wires 15a and 15b, and that can be moved together with the wires 15a and 15b, so as to detect the actual bending angle of the bending section 15 on the basis of the movement amounts of the linear members. In that case, the linear members correspond to sensors.

Alternatively, the bending-section-shape detecting part 24 may detect the bending shape of the bending section 15 by using an inserted-endoscope-shape observation device. In that case, a plurality of magnetic coils are installed in the bending section 15 at different positions in the longitudinal direction. The inserted-endoscope-shape observation device receives magnetism produced from the magnetic coils installed in the bending section 15, calculates the positions of the magnetic coils from the received magnetism, and connects the obtained positions of the magnetic coils with a smooth curve, thus obtaining the bending shape of the bending section 15.

In this embodiment, as shown in Fig. 11, the control unit 3 has a table in which the channels 5A and 5B and the treatment tools 6 are associated with FF gains $K_i + f_\theta + f_R$ (wherein, i = 1, 2, 3, or 4). The FF gain is defined as a function $f_\theta$ of the bending angle θ of the bending section 15 and a function $f_R$ of a bending radius R of the channel 5A or 5B at the bending angle θ. As described in the second embodiment, the control unit 3 selects one of the four FF gains $K_i + f_\theta + f_R$ and substitutes the bending angle θ received from the bending-section-shape detecting part 24 and the bending radius R of the channel 5A or 5B corresponding to the bending angle θ into the functions $f_\theta$ and $f_R$ for the selected FF gain, thereby calculating the value of the FF gain.

Next, the operation of the thus-configured manipulator system 300 will be described.

The basic procedure is the same as that in the first embodiment. The manipulator system 300 of this embodiment differs from those of the first and second embodiments in the FF-gain setting method.

As described in the second embodiment, the control unit 3 selects the FF gain $K_i + f_\theta + f_R$ according to the combination of the treatment tool 6 and the channel 5A or 5B into which the treatment tool 6 has been inserted. After that, when an operation for the treatment tool 6 is input to the master arms 9, the control unit 3 receives the bending angle θ of the bending section 15 from the bending-section-shape detecting part 24, calculates the value of the FF gain by using the received bending angle θ and the bending radius R of the channel 5A or 5B corresponding thereto, multiplies the forward/backward control signal and the rotary control signal by the calculated value, and sends the amplified control signals to the treatment-tool driving part 13.

Here, the variation in the above-described responsiveness of the treatment tool 6 additionally depends on the bending angle $\theta$ of the bending section 15 and the bending radius R of each of the channels 5A and 5B. For example, as shown in Fig. 12, when the bending section 15 is bent such that the first channel 5A is located on an outer circumferential side, a bending radius R1 of the first channel 5A and a bending radius R2 of the second channel 5B are different from each other, and R1 > R2 is established. Therefore, an appropriate FF gain varies depending on the bending angle $\theta$ of the bending section 15 and also depending on whether the treatment tool 6 is inserted into the channel 5A or 5B.

According to this embodiment, the FF gain $K_i + f_\theta + f_R$ is set depending, in addition, on the bending angle $\theta$ of the bending section 15 and the bending radius R of the channel 5A or 5B, thereby further compensating for the variation in the movement responsiveness of the treatment tool 6, which is caused by the difference in the bending angle $\theta$ of the bending section 15 and in the bending radius R. Accordingly, in addition to the advantageous effect of the second embodiment, there is the advantage that, regardless of the bending angle $\theta$ at which the treatment tool 6 is used, it is possible to always obtain superior, uniform responsiveness of the forward/backward movement and the rotary movement of the treatment tool 6.

Note that, in this embodiment, the FF gain may be set depending, in addition, on the bending direction of the bending section 15.

For example, a different function $f_\theta$ may be set depending on whether the bending section 15 is bent toward the left (specifically, $-90° \leq \theta \leq 0°$) or right (specifically, $0 \leq \theta \leq 90°$).

As shown in Fig. 13, the bending radius R (R1 or R2) of the corresponding channel 5A or 5B varies depending on whether the bending section 15 is bent toward the left or right. Thus, the function $f_\theta$ may be set for each bending direction of the bending section 15 to modify the FF gains shown in Fig. 11 as in the following equations. Here, K is $K_1$, $K_2$, $K_3$, or $K_4$.

$$\texttt{FF gain (left)} = K + f_{\theta\_LEFT} + f_R$$

$$\texttt{FF gain (right)} = K + f_{\theta\_RIGHT} + f_R$$

By doing so, the responsiveness of the treatment tool 6 can be further improved.

Furthermore, in this embodiment, it is also possible to reflect, in the compensation value, the bending angles of the bending section 15 in the LR direction and in the UD direction, to modify the FF gains shown in Fig. 11 as in the following equation. Here, $f_{\theta\_LR}$ is a function of a bending angle $\theta_{LR}$ of the bending section 15 in the LR direction, $f_{R\_LR}$ is a function of the bending radius of each channel 5A or 5B in a plane LR at the bending angle $\theta_{LR}$, $f_{\theta\_UD}$ is a function of a bending angle $\theta_{UD}$ of the bending section 15 in the UD direction, and $f_{R\_UD}$ is a function of the bending radius of each channel 5A or 5B in a plane UD at the bending angle $\theta_{UD}$.

$$\texttt{FF gain} = K + f_{\theta\_LR} + f_{R\_LR} + f_{\theta\_UD} + f_{R\_UD}$$

In a state in which the bending section 15 is bent in both the LR direction and the UD direction, the bending shapes of the channels 5A and 5B are affected by both the bending angle of the bending section 15 in the LR direction and the bending angle of the bending section 15 in the UD direction. Therefore, by using the above equation, the responsiveness of the treatment tool 6 can be further improved.

Furthermore, in this embodiment, the functions $f_{\theta\_LR}$, $f_{R\_LR}$, $f_{\theta\_UD}$, and $f_{R\_UD}$ may be set optimally for each of the channels 5A and 5B, to modify the FF gains shown in Fig. 11 as in the following equations.

$$\text{FF gain (first channel)}$$
$$= K + f_{\theta\_LR1} + f_{R\_LR1} + f_{\theta\_UD1} + f_{R\_UD1}$$

$$\text{FF gain (second channel)}$$
$$= K + f_{\theta\_LR2} + f_{R\_LR2} + f_{\theta\_UD2} + f_{R\_UD2}$$

As shown in Fig. 14, when the direction of arrangement of the channels 5A and 5B is inclined with respect to the bending directions (the LR direction and the UD direction) of the bending section 15, the relationships between the

bending angles $\theta_{LR}$ and $\theta_{UD}$ of the bending section 15 and the bending radii R of each channel 5A or 5B in the LR direction and the UD direction vary. Therefore, by using the above equations, the responsiveness of the treatment tool 6 can be further improved.

Fourth Embodiment

Next, a manipulator system 400 according to a fourth embodiment of the present invention will be described with reference to Figs. 15 to 17.

As shown in Fig. 15, the manipulator system 400 of this embodiment is obtained by further providing, in the manipulator system 300 of the third embodiment, a flexible-section-shape detecting part 25 that detects the bending shape of the flexible section 14. Therefore, in this embodiment, the flexible-section-shape detecting part 25 will be mainly described, identical reference signs will be assigned to the same configurations as those in the first to third embodiments, and a description thereof will be omitted.

The flexible-section-shape detecting part 25 is provided with the above-described inserted-endoscope-shape observation device. Specifically, the flexible-section-shape detecting part 25 receives magnetism produced from a plurality of magnetic coils installed in the flexible section 14 at different positions in the longitudinal direction, calculates the positions of the magnetic coils from the received magnetism, and connects the obtained positions of the magnetic coils with a smooth curve, thus obtaining the bending shape of the flexible section 14.

Next, the flexible-section-shape detecting part 25 calculates, from the bending shape of the flexible section 14 obtained by the inserted-endoscope-shape observation device, a feature quantity k that indicates the bending amount of the entire flexible section 14, according to the following procedure. First, as shown in Figs. 16A and 16B, the flexible-section-shape detecting part 25 divides the flexible section 14 into small segments $\Delta d$ in the longitudinal direction and measures a bending radius r and a bending angle $\Delta \phi$ for each of the small segments $\Delta d$. The flexible-section-shape detecting part 25 holds a function that defines the relationship between a feature quantity $\Delta k$ of the small segment $\Delta d$, and the bending radius r and the bending angle $\Delta \phi$ thereof. This function is set such that the feature quantity $\Delta k$ is zero when the flexible section 14 linearly extends (specifically, when $r = \infty$, and when the bending angle $\Delta \phi = 0$), and the feature quantity $\Delta k$ increases as the bending radius r becomes smaller and as the bending angle $\Delta \phi$ becomes larger. By substituting the bending radius r and the bending angle $\Delta \phi$ into this function, the feature quantity $\Delta k$ of each small segment $\Delta d$ can be obtained. Then, the feature quantities $\Delta k$ over the entire length of the flexible section 14 are integrated, thereby calculating the feature quantity k, which indicates the bending amount of the bending shape of the entire flexible section 14. In Fig. 16A, reference sign X denotes the large intestine into which the insertion portion 5 is inserted.

In this embodiment, as shown in Fig. 17, the control unit 3 has a table in which the channels 5A and 5B and the treatment tools 6 are associated with FF gains $K_i + f_\theta + f_R + g_k$ (wherein, i = 1, 2, 3, or 4). The FF gain is further defined as a function $g_k$ of the feature quantity k of the flexible section 14. As described in the second embodiment, the control unit 3 selects one of the four FF gains $K_i + f_\theta + f_R + g_k$ and substitutes, into the functions $f_\theta$, $f_R$, and $g_k$ for the selected FF gain, the bending angle $\theta$, the bending radius R, and further the feature quantity k, which is received from the flexible-section-shape detecting part 25, thereby calculating the value of the FF gain.

Next, the operation of the thus-configured manipulator system 400 will be described.

The basic procedure is the same as that in the first embodiment. The manipulator system 400 of this embodiment differs from those of the first to third embodiments in the FF-gain setting method.

As described in the second embodiment, the control unit 3 selects the FF gain $K_i + f_\theta + f_R + g_k$ according to the combination of the treatment tool 6 and the channel 5A or 5B into which the treatment tool 6 has been inserted. After that, when an operation for the treatment tool 6 is input to the master arms 9, the control unit 3 further receives the feature quantity k from the flexible-section-shape detecting part 25, calculates the value of the FF gain by using the received feature quantity k and the above-described bending angle $\theta$ and bending radius R, multiplies the forward/backward control signal and the rotary control signal by the calculated value, and sends the amplified control signals to the treatment-tool driving part 13.

Here, the variation in the above-described responsiveness of the treatment tool 6 additionally depends on the bending shape of the flexible section 14. For example, the movement responsiveness of the treatment tool 6 when the flexible section 14 is bent is lower than when the flexible section 14 linearly extends. Such a reduction and a variation in the movement responsiveness of the treatment tool 6 are caused because the friction and the slack produced in the main body 18 of the treatment tool 6 in the channel 5A or 5B vary depending on the bending shape of the flexible section 14.

According to this embodiment, the FF gain $K_i + f_\theta + f_R + g_k$ is set depending, in addition, on the bending shape of the flexible section 14, thereby further compensating for the variation in the movement responsiveness of the treatment tool 6, which is caused by the difference in the bending shape of the flexible section 14. Accordingly, in addition to the advantageous effect of the third embodiment, there is the advantage that, regardless of the bending shape of the flexible section 14 in which the treatment tool 6 is used, it is possible to always obtain superior, uniform responsiveness of the forward/backward movement and the rotary movement of the treatment tool 6.

Note that, in this embodiment, it is preferred that the tubes, which form the channels 5A and 5B, be fixed in position, in radial direction, in the insertion portion 5.

If each of the tubes can be radially moved in the flexible section 14 and the bending section 15, the optimal compensation value may possibly vary even in the same bending shape of the flexible section 14 and the bending section 15. This is because, if a variation is caused in the tube pathway, this may possibly cause variations in the friction and in the slack produced in the main body 18 of the treatment tool 6.

Thus, by defining the tube pathway in the insertion portion 5, the bending shape of the flexible section 14 and the bending section 15 and the bending shape of the main body 18 of the treatment tool 6 are associated on a one-to-one basis; therefore, it is possible to improve the accuracy of compensation of the reduction and the variation in the responsiveness of the treatment tool 6 using the compensation value.

Fifth Embodiment

Next, a manipulator system 500 according to a fifth embodiment of the present invention will be described with reference to Figs. 18 and 19.

As shown in Fig. 18, the manipulator system 500 of this embodiment is obtained by further providing, in the manipulator system 400 of the fourth embodiment, an external-section-shape detecting part 26 that detects the bending shape of a base-end portion of the main body 18 of the treatment tool 6 by identifying the attachment unit 20 on the treatment tool 6 that has been inserted into the insertion portion 5. Therefore, in this embodiment, the external-section-shape detecting part 26 will be mainly described, identical reference signs will be assigned to the same configurations as those in the first to fourth embodiments, and a description thereof will be omitted.

The external-section-shape detecting part 26 detects a bending angle $\theta_{ex}$ of the base-end portion of the main body 18, the base-end portion being pulled out to the outside of the insertion portion 5 from the treatment-tool port 17 (hereinafter, this portion is referred to as an external portion of the treatment tool 6).

Because the position of the treatment-tool attachment part 11A or 11B to which the attachment unit 20 is attached is different depending on whether the treatment tool 6 is inserted into the channel 5A or 5B, the pulled shape of the external portion of the treatment tool 6 varies. Furthermore, the pulled shape varies for each treatment tool 6 depending, in addition, on the stiffness, the length, etc., of the main body 18. The pulled shape of the external portion is almost fixed for each combination of the treatment-tool attachment part 11A or 11B and the treatment tool 6. Therefore, by measuring the bending angle $\theta_{ex}$ of the external portion of each treatment tool 6 when the attachment unit 20 is attached to each of the treatment-tool attachment parts 11A and 11B, a table in which the treatment tool 6 and the treatment-tool attachment part 11A or 11B are associated with the bending angle $\theta_{ex}$ is obtained in advance. Then, the external-section-shape detecting part 26 can detect the bending angle $\theta_{ex}$ by referring to the table on the basis of the above-described identification information of the channel 5A or 5B and the treatment tool 6.

In this embodiment, as shown in Fig. 19, the control unit 3 has a table in which the channels 5A and 5B and the treatment tools 6 are associated with FF gains $K_i + f_\theta + f_R + g_k + h_{\theta ex}$ (wherein, i = 1, 2, 3, or 4). The FF gain is further defined as a function $h_{\theta ex}$ of the bending angle $\theta_{ex}$ of the external portion. As described in the second embodiment, the control unit 3 selects one of the four FF gains $K_i + f_\theta + f_R + g_k + h_{\theta ex}$, and substitutes the bending angle $\theta$, the bending radius R, the feature quantity k, and the bending angle $\theta_{ex}$, which is received from the external-section-shape detecting part 26, into the functions $f_\theta$, $f_R$, $g_k$, and $h_{\theta ex}$ for the selected FF gain, thereby calculating the value of the FF gain.

Next, the operation of the thus-configured manipulator system 500 will be described.

The basic procedure is the same as that of the first embodiment. The manipulator system 500 of this embodiment differs from those of the first to fourth embodiments in the FF-gain setting method.

As described in the second embodiment, the control unit 3 selects the FF gain $K_i + f_\theta + f_R + g_k + h_{\theta ex}$ according to the combination of the treatment tool 6 and the channel 5A or 5B into which the treatment tool 6 has been inserted. After that, when an operation for the treatment tool 6 is input to the master arms 9, the control unit 3 further receives the bending angle $h_{\theta ex}$ from the external-section-shape detecting part 26, calculates the value of the FF gain by using the received bending angle $h_{\theta ex}$ and the above-described bending angle $\theta$, bending radius R, and feature quantity k, multiplies the forward/backward control signal and the rotary control signal by the calculated value, and sends the amplified control signals to the treatment-tool driving part 13.

Here, the variation in the above-described responsiveness of the treatment tool 6 additionally depends on the bending shape of the external portion of the treatment tool 6.

According to this embodiment, the FF gain $K_i + f_\theta + f_R + g_k + h_{\theta ex}$ is set depending, in addition, on the bending angle $\theta_{ex}$ of the external portion, thereby further compensating for the variation in the movement responsiveness of the treatment tool 6, which is caused by the difference in the bending angle $\theta_{ex}$ of the external portion. Accordingly, in addition to the advantageous effect of the fourth embodiment, there is the advantage that, regardless of the pulled pathway of the external portion in which the treatment tool 6 is used, it is possible to always obtain superior, uniform responsiveness of the forward/backward movement and the rotary movement of the treatment tool 6.

Sixth Embodiment

Next, a manipulator system 600 according to a sixth embodiment of the present invention will be described with reference to Figs. 20 to 22.

As shown in Fig. 20, the manipulator system 600 of this embodiment is obtained by further providing, in the manipulator system 300 of the third embodiment, two bending-section-shape detecting parts 242 and 243. Therefore, in this embodiment, the bending-section-shape detecting parts 242 and 243 will be mainly described, identical reference signs will be assigned to the same configurations as those in the first to fifth embodiments, and a description thereof will be omitted.

In this embodiment, as shown in Fig. 21, the insertion portion 5 is further provided with two bending sections 152 and 153. Specifically, two arm parts 142 and 143 that extend from the distal end of the insertion portion 5 are provided, and the arm parts 142 and 143 have the bending sections 152 and 153 at distal end portions thereof. The channels 5A and 5B extend up to the distal ends of the arm parts 142 and 143, and the treatment tools 6 can protrude from the distal ends of the arm parts 142 and 143. Like the bending section 15, the bending sections 152 and 153 are driven by bending-section driving parts (not shown) provided therefor.

The bending-section-shape detecting parts 242 and 243 detect bending angles $\theta2$ and $\theta3$ of the bending sections 152 and 153, respectively, in the same way as the bending-section-shape detecting part 24, which is described in the third embodiment.

In this embodiment, as shown in Fig. 22, the control unit 3 has a table in which the channels 5A and 5B and the treatment tools 6 are associated with FF gains $K_i + f_\theta + f_R + f_{\theta2 \text{ or } \theta3} + f_{R2 \text{ or } R3}$ (wherein, i = 1, 2, 3, or 4). Here, $f_{\theta2 \text{ or } \theta3}$ is a function of the bending angle $\theta2$ or $\theta3$ of the corresponding bending section 152 or 153, and $f_{R2 \text{ or } R3}$ is a function of the bending radius R2 or R3 of the corresponding channel 5A or 5B at the bending angle $\theta2$ or $\theta3$.

The control unit 3 further substitutes, into the functions $f_{\theta2 \text{ or } \theta3}$ and $f_{R2 \text{ or } R3}$ for the FF gain, the bending angle $\theta2$ or $\theta3$ received from the bending-section-shape detecting part 242 or 243 and the bending radius R2 or R3 of the channel 5A or 5B corresponding to the bending angle $\theta2$ or $\theta3$, thereby calculating the value of the FF gain.

The operation of the manipulator system 600 of this embodiment is the same as that of the third embodiment except that the bending angle $\theta2$ or $\theta3$ of the bending section 152 or 153 of the arm part 142 or 143 into which the treatment tool 6 has been inserted and the bending radius R2 or R3 are further used to calculate the value of the FF gain.

According to this embodiment, in a case in which the arm parts 142 and 143, which have the bending sections 152 and 153, are connected to the distal end of the flexible section 14, and the channels 5A and 5B are bent at two positions, the FF gain is set according to the bending angles and the bending radii of both bending sections 15 and 152 or 15 and 153, thereby making it possible to more accurately compensate for the variation in the movement responsiveness of the treatment tool 6.

Note that, in the above-described embodiments and modifications thereof, a description has been given of an example configuration in which the manipulator 1 and the operation input unit 2 are separately provided, and the operation input unit 2, which is disposed at a position away from the manipulator 1, remotely controls the manipulator 1; however, the arrangement of the manipulator 1 and the operation input unit 2 is not limited thereto, and, for example, the operation input unit 2 may be integrally disposed at a rear end of the manipulator 1.

{Reference Signs List}

1 slave manipulator (manipulator)
2 master input unit (operation input unit)
3 control unit (compensation-value setting unit)
4 slave arm
5 insertion portion
5A, 5B channel
6 treatment tool
7 observation member
8 display unit
9 master arm
10 insertion-portion attachment part
11A, 11B treatment-tool attachment part
12 bending-section driving part

12a motor

13A, 13B treatment-tool driving part

13a, 13b motor

14 flexible section

15, 152, 153 bending section

15a, 15b wire (linear member)

16, 20 attachment unit

17 treatment-tool port

18 main body

19 treatment part

22 channel-in-use detecting part

23 treatment-tool identifying part

24, 242, 243 bending-section-shape detecting part

25 flexible-section-shape detecting part

26 external-section-shape detecting part

80 operating table

100, 200, 300, 400, 500, 600 manipulator system

142, 143 arm part

Op operator

**Claims**

1. A manipulator system comprising:

   a manipulator that includes an elongated insertion portion having a plurality of channels formed therethrough in the longitudinal direction, a treatment tool to be inserted into one of the channels of the insertion portion, and a treatment-tool driving part that allows the treatment tool to perform at least one of a forward/backward movement and a rotary movement in the channel;
   an operation input unit to which an operator inputs an operation instruction for the treatment tool;
   a control unit that generates a control signal for driving the treatment-tool driving part, according to the operation instruction input to the operation input unit;
   a channel-in-use detecting part that detects, among the plurality of channels, the channel into which the treatment tool has been inserted; and
   a compensation-value setting unit that sets a compensation value for the control signal on the basis of the channel detected by the channel-in-use detecting part,
   wherein the control unit compensates the control signal by using the compensation value set by the compensation-value setting unit and sends the compensated control signal to the treatment-tool driving part.

2. The manipulator system according to claim 1, further comprising a treatment-tool identifying part that identifies the treatment tool that has been inserted into the channel,
   wherein the compensation-value setting unit sets the compensation value additionally on the basis of a dynamic property of the treatment tool identified by the treatment-tool identifying part.

3. The manipulator system according to claim 1 or 2,
   wherein the insertion portion includes an elongated flexible section that has flexibility and a bendable bending section that is provided at a distal end of the flexible section;
   the manipulator system further comprises a bending-section-shape detecting part that detects a bending shape of the bending section; and
   the compensation-value setting unit sets the compensation value additionally on the basis of the bending shape of the bending section detected by the bending-section-shape detecting part.

4. The manipulator system according to one of claims 1 to 3,
   wherein the insertion portion includes an elongated flexible section that has flexibility and a bendable bending section that is provided at a distal end of the flexible section;
   the manipulator system further comprises a flexible-section-shape detecting part that detects a bending shape of the flexible section; and
   the compensation-value setting unit sets the compensation value additionally on the basis of the bending shape of the flexible section detected by the flexible-section-shape detecting part.

5. The manipulator system according to one of claims 1 to 4, further comprising an external-section-shape detecting part that detects a bending shape of a base-end portion of the treatment tool, the base-end portion being pulled out from the insertion portion to the outside,
wherein the compensation-value setting unit sets the compensation value additionally on the basis of the bending shape of the base-end portion of the treatment tool detected by the external-section-shape detecting part.

6. The manipulator system according to one of claims 1 to 5,
wherein the channels are made of tubes that are disposed in the insertion portion along the longitudinal direction; and the tubes are fixed in position, in radial position, in the insertion portion.

7. The manipulator system according to one of claims 1 to 6,
wherein the control unit performs feedforward control for the treatment-tool driving part; and
the compensation-value setting unit sets, as the compensation value, a gain that is used for the feedforward control by the control unit.

8. The manipulator system according to one of claims 1 to 6,
wherein the control unit performs feedback control for the treatment-tool driving part; and
the compensation-value setting unit sets, as the compensation value, a gain that is used for the feedback control by the control unit.

9. The manipulator system according to one of claims 1 to 6,
wherein the control unit sends, as the control signal, a control signal on which an offset signal is superimposed, to the treatment-tool driving part; and
the compensation-value setting unit sets, as the compensation value, the offset signal, whose sign is reversed when the direction of movement of the treatment tool is switched to the reverse direction.


**Patentansprüche**

1. Manipulatorsystem, umfassend:

einen Manipulator, der einen länglichen Einsetzabschnitt mit einer Vielzahl von darin gebildeten Kanälen in der Längsrichtung, ein Behandlungswerkzeug, das in einen der Kanäle des Einsetzabschnitts einzusetzen ist, und ein
Behandlungswerkzeugantriebsteil umfasst, das es ermöglicht, dass das Behandlungswerkzeug mindestens eins von einer Vorwärts-/Rückwärtsbewegung und einer Drehbewegung in dem Kanal durchführt;
eine Bedieneingabeeinheit, in welche ein Bediener eine Bedienanweisung für das Behandlungswerkzeug eingibt;
eine Steuerungseinheit, die ein Steuerungssignal zum Antreiben des Behandlungswerkzeugantriebsteils gemäß der Bedieneinweisungseingabe in die Bedieneingabeeinheit erzeugt,
ein Kanal-in-Verwendung-Erfassungsteil, das unter der Vielzahl von Kanälen den Kanal erfasst, in den das Behandlungswerkzeug eingesetzt wurde; und
eine Kompensationswerteinstelleinheit, die einen Kompensationswert für das Steuerungssignal auf der Basis des von dem Kanal-in-Verwendung-Erfassungsteil erfassten Kanals einstellt,
wobei die Steuerungseinheit das Steuerungssignal mithilfe des von der Kompensationswerteinstelleinheit eingestellten Kompensationswerts kompensiert und das kompensierte Steuerungssignal an den Behandlungswerkzeugantriebsteil sendet.

2. Manipulatorsystem nach Anspruch 1, das ferner ein Behandlungswerkzeugidentifizierungsteil umfasst, das das Behandlungswerkzeug, das in den Kanal eingesetzt wurde, identifiziert,
wobei die Kompensationswerteinstelleinheit den Kompensationswert zusätzlich auf der Basis einer dynamischen Eigenschaft des von dem Behandlungswerkzeugidentifizierungsteil identifizierten Behandlungswerkzeugs einstellt.

3. Manipulatorsystem nach Anspruch 1 oder 2,
wobei der Einsetzabschnitt einen länglichen flexiblen Abschnitt mit Flexibilität und einen biegbaren Biegeabschnitt umfasst, der an einem distalen Ende des flexiblen Abschnitts vorgesehen ist;

das Manipulatorsystem ferner ein Biegeabschnittformerfassungsteil umfasst, das eine Biegeform des Biegeabschnitts erfasst; und

die Kompensationswerteinstelleinheit den Kompensationswert zusätzlich auf der Basis der Biegeform des von dem Biegeabschnittformerfassungsteil erfassten Biegeabschnitts einstellt.

4. Manipulatorsystem nach einem der Ansprüche 1 bis 3,
wobei der Einsetzabschnitt einen länglichen flexiblen Abschnitt mit Flexibilität und einen biegbaren Biegeabschnitt umfasst, der an einem distalen Ende des flexiblen Abschnitts vorgesehen ist;
das Manipulatorsystem ferner ein Formerfassungsteil für den flexiblen Abschnitt umfasst, das eine Biegeform des flexiblen Abschnitts erfasst; und
die Kompensationswerteinstelleinheit den Kompensationswert zusätzlich auf der Basis der Biegeform des von dem Formerfassungsteil für den flexiblen Abschnitt erfassten flexiblen Abschnitts einstellt.

5. Manipulatorsystem nach einem der Ansprüche 1 bis 4, das ferner ein Formerfassungsteil für den externen Abschnitt umfasst, das eine Biegeform eines Basisendabschnitts des Behandlungswerkzeugs erfasst, wobei der Basisendabschnitt aus dem Einsetzabschnitt zur Außenseite herausgezogen wird,
wobei die Kompensationswerteinstelleinheit den Kompensationswert zusätzlich auf der Basis der Biegeform des von dem Formerfassungsteil für den externen Abschnitt erfassten Basisendabschnitts des Behandlungswerkzeugs einstellt.

6. Manipulatorsystem nach einem der Ansprüche 1 bis 5, wobei die Kanäle aus Rohren bestehen, die in dem Einsetzabschnitt entlang der Längsrichtung angeordnet sind; und die Rohre in radialer Position, in dem Einsetzabschnitt in Position fixiert sind.

7. Manipulatorsystem nach einem der Ansprüche 1 bis 6, wobei die Steuerungseinheit eine Vorschubsteuerung für das Behandlungswerkzeugantriebsteil durchführt; und
die Kompensationswerteinstelleinheit als Kompensationswert eine Verstärkung einsetzt, die zur Vorschubsteuerung von der Steuerungseinheit genutzt wird.

8. Manipulatorsystem nach einem der Ansprüche 1 bis 6, wobei die Steuerungseinheit eine Rückschubsteuerung für das Behandlungswerkzeugantriebsteil durchführt; und
die Kompensationswerteinstelleinheit als Kompensationswert eine Verstärkung einsetzt, die zur Rückschubsteuerung von der Steuerungseinheit genutzt wird.

9. Manipulatorsystem nach einem der Ansprüche 1 bis 6, wobei die Steuerungseinheit als Steuerungssignal ein Steuerungssignal, das mit einem Versatzsignal überlagert ist, an das Behandlungswerkzeugantriebsteil sendet; und
die Kompensationswerteinstelleinheit als Kompensationswert das Versatzsignal einstellt, dessen Zeichen umgekehrt wird, wenn die Richtung der Bewegung des Behandlungswerkzeugs auf die umgekehrte Richtung geschaltet wird.

**Revendications**

1. Système de manipulateur comprenant :

un manipulateur qui comprend une partie d'insertion allongée ayant une pluralité de canaux formés à travers celle-ci dans la direction longitudinale, un outil de traitement à insérer dans l'un des canaux de la partie d'insertion, et une partie d'entraînement d'outil de traitement qui permet à l'outil de traitement de réaliser au moins un parmi un mouvement vers l'avant/vers l'arrière et un mouvement de rotation dans le canal ;
une unité d'entrée d'opération dans laquelle un opérateur entre une instruction d'opération pour l'outil de traitement ;
une unité de commande qui génère un signal de commande pour entraîner la partie d'entraînement d'outil de traitement, selon l'instruction d'opération entrée dans l'unité d'entrée d'opération ;
une partie de détection de canal en utilisation qui détecte, parmi la pluralité de canaux, le canal dans lequel l'outil de traitement a été inséré ; et
une unité de réglage de valeur de compensation qui règle une valeur de compensation pour le signal de commande sur la base du canal détecté par la partie de détection de canal en utilisation,
l'unité de commande compensant le signal de commande à l'aide de la valeur de compensation réglée par l'unité de réglage de valeur de compensation et envoyant le signal de commande compensé à la partie d'entraînement d'outil de traitement.

**2.** Système de manipulateur selon la revendication 1, comprenant en outre une partie d'identification d'outil de traitement qui identifie l'outil de traitement qui a été inséré dans le canal,
l'unité de réglage de valeur de compensation réglant la valeur de compensation également sur la base d'une propriété dynamique de l'outil de traitement identifié par la partie d'identification d'outil de traitement.

**3.** Système de manipulateur selon la revendication 1 ou 2,
dans lequel la partie d'insertion comprend une section souple allongée qui a une flexibilité et une section de courbure apte à être courbée qui est prévue à une extrémité distale de la section souple ;
le système de manipulateur comprenant en outre une partie de détection de forme de section de courbure qui détecte une forme de courbure de la section de courbure ; et
l'unité de réglage de valeur de compensation réglant la valeur de compensation également sur la base de la forme de courbure de la section de courbure détectée par la partie de détection de forme de section de courbure.

**4.** Système de manipulateur selon l'une des revendications 1 à 3,
dans lequel la partie d'insertion comprend une section souple allongée qui a une flexibilité et une section de courbure apte à être courbée qui est prévue à une extrémité distale de la section souple ;
le système de manipulateur comprenant en outre une partie de détection de forme de section souple qui détecte une forme de courbure de la section souple ; et
l'unité de réglage de valeur de compensation réglant la valeur de compensation également sur la base de la forme de courbure de la section souple détectée par la partie de détection de forme de section souple.

**5.** Système de manipulateur selon l'une des revendications 1 à 4, comprenant en outre une partie de détection de forme de section externe qui détecte une forme de courbure d'une partie d'extrémité de base de l'outil de traitement, la partie d'extrémité de base étant retirée de la partie d'insertion vers l'extérieur,
l'unité de réglage de valeur de compensation réglant la valeur de compensation également sur la base de la forme de courbure de la partie d'extrémité de base de l'outil de traitement détectée par la partie de détection de forme de section externe.

**6.** Système de manipulateur selon l'une des revendications 1 à 5,
dans lequel les canaux sont faits de tubes qui sont disposés dans la partie d'insertion le long de la direction longitudinale ; et
les tubes étant fixés en position, en position radiale, dans la partie d'insertion.

**7.** Système de manipulateur selon l'une des revendications 1 à 6,
dans lequel l'unité de commande réalise une commande prédictive pour la partie d'entraînement d'outil de traitement ; et
l'unité de réglage de valeur de compensation réglant, en tant que valeur de compensation, un gain qui est utilisé pour la commande prédictive par l'unité de commande.

**8.** Système de manipulateur selon l'une des revendications 1 à 6,
dans lequel l'unité de commande réalise une commande à rétroaction pour la partie d'entraînement d'outil de traitement ; et
l'unité de réglage de valeur de compensation réglant, en tant que valeur de compensation, un gain qui est utilisé pour la commande à rétroaction par l'unité de commande.

**9.** Système de manipulateur selon l'une des revendications 1 à 6,
dans lequel l'unité de commande envoie, en tant que signal de commande, un signal de commande sur lequel un signal de décalage est superposé, à la partie d'entraînement d'outil de traitement ; et
l'unité de réglage de valeur de compensation réglant, en tant que valeur de compensation, le signal de décalage, dont le signe est inversé lorsque la direction de mouvement de l'outil de traitement est changée dans la direction inverse.

FIG. 1

EP 3 025 675 B1

CONTROL UNIT

# FIG. 2

EP 3 025 675 B1

FIG. 3

EP 3 025 675 B1

FIG. 4

EP 3 025 675 B1

# FIG. 5

# FIG. 6

# FIG. 7

EP 3 025 675 B1

FIG. 8

EP 3 025 675 B1

# FIG. 9

| | FIRST CHANNEL | SECOND CHANNEL |
|---|---|---|
| FIRST TREATMENT TOOL | $K_1$ | $K_2$ |
| SECOND TREATMENT TOOL | $K_3$ | $K_4$ |

FIG. 10

EP 3 025 675 B1

# FIG. 11

|  | FIRST CHANNEL | SECOND CHANNEL |
|---|---|---|
| FIRST TREATMENT TOOL | $K_1 + f_\theta + f_R$ | $K_2 + f_\theta + f_R$ |
| SECOND TREATMENT TOOL | $K_3 + f_\theta + f_R$ | $K_1 + f_\theta + f_R$ |

FIG. 12

FIG. 13

# FIG. 14

FIG. 15

EP 3 025 675 B1

## FIG. 16A

## FIG. 16B

# FIG. 17

| | FIRST CHANNEL | SECOND CHANNEL |
|---|---|---|
| FIRST TREATMENT TOOL | $K_1 + f_\theta + f_R + g_k$ | $K_2 + f_\theta + f_R + g_k$ |
| SECOND TREATMENT TOOL | $K_3 + f_\theta + f_R + g_k$ | $K_4 + f_\theta + f_R + g_k$ |

FIG. 18

EP 3 025 675 B1

## FIG. 19

| | FIRST CHANNEL | SECOND CHANNEL |
|---|---|---|
| FIRST TREATMENT TOOL | $K_1 + f_\theta + f_R + g_k + h_{\theta ex}$ | $K_2 + f_\theta + f_R + g_k + h_{\theta ex}$ |
| SECOND TREATMENT TOOL | $K_3 + f_\theta + f_R + g_k + h_{\theta ex}$ | $K_4 + f_\theta + f_R + g_k + h_{\theta ex}$ |

EP 3 025 675 B1

FIG. 20

EP 3 025 675 B1

FIG. 21

EP 3 025 675 B1

# FIG. 22

| | FIRST CHANNEL | SECOND CHANNEL |
|---|---|---|
| FIRST TREATMENT TOOL | $K_1 + f_\theta + f_R + f_{\theta 2} + f_{R2}$ | $K_2 + f_\theta + f_R + f_{\theta 3} + f_{R3}$ |
| SECOND TREATMENT TOOL | $K_3 + f_\theta + f_R + f_{\theta 2} + f_{R2}$ | $K_4 + f_\theta + f_R + f_{\theta 3} + f_{R3}$ |

EP 3 025 675 B1

**EP 3 025 675 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007089808 A
- WO 2010151438 A
- US 2008051629 A
- JP 2008245840 B